# FASCICULE DE BREVET EUROPEEN

(11) **EP 0 679 383 B1**
(45) Date de publication et mention de la délivrance du brevet: **04.09.2002**
(21) Numéro de dépôt: 95400444.6
(22) Date de dépôt: 01.03.1995
(51) Int. Cl.: A61K 7/04, A61K 7/043

(54) **Procédé de soin des ongles**
Verfahren zur Pflege der Nägel
Nail care method

(30) Priorité: 15.04.1994 FR 9404543
(43) Date de publication de la demande: 02.11.1995
(73) Titulaire: L'OREAL, 75008 Paris (FR)
(72) Inventeur: Ramin, Roland, F-91760 Itteville (FR)
(74) Mandataire: Dodin, Catherine

(56) Documents cités:
- US-A- 5 102 654

## Description

La présente invention a trait à un procédé de soin de l'ongle.

Il est connu de préparer des vernis à ongles à partir, par exemple, d'un mélange de polymère, de plastifiant et d'agents rhéologiques en solution organique. On obtient ainsi des vernis facilement applicables sur l'ongle avec une couvrance et une adhésion relativement bonnes, dépendant de la nature de l'association polymère/plastifiant utilisée.
Il est également connu d'incorporer à ces vernis, contenant ou non des pigments colorés, des agents actifs tels que des durcisseurs de manière à obtenir des bases de soin pour ongle applicables en tant que sous-couche d'un vernis solvant ou en tant que telles.
Or, on a constaté que, si les bases ainsi obtenues ont éventuellement une action sur l'ongle, à savoir par exemple le durcir, elles ne permettent pas une éventuelle modification et/ou amélioration de sa structure.
Ainsi, si l'on applique de telles bases sur un ongle fragile, par exemple un ongle mou qui a tendance à se dédoubler, on peut obtenir un ongle plus dur mais qui aura toujours tendance à se fendiller et à se casser.
Il est connu du document US-A-5,102,654 un vernis à ongles sous forme d'émulsion contenant une phase aqueuse et une phase laque et pouvant comprendre comme émollient du phytantriol.
La présente invention a pour but de proposer un procédé de soin des ongles permettant à la fois d'améliorer l'aspect extérieur de l'ongle, par exemple en l'hydratant, le nourrissant, le durcissant, tout en le restructurant c'est-à-dire en diminuant sa faculté à se dédoubler et/ou se fendiller.

Un objet de la présente invention est donc un procédé de soin des ongles selon la revendication 1.
Un autre objet de l'invention est l'utilisation du phytantriol en tant qu'agent restructurant dans une composition de soin pour ongles se présentant sous la forme d'une solution organique.

On a constaté que de manière surprenante l'utilisation de phytantriol dans un vernis ou une base pour l'ongle permet d'obtenir une composition de soin susceptible, après application pendant un certain temps, de restructurer l'ongle en réduisant sa faculté à se dédoubler. Dans la présente composition du procédé selon l'invention, le phytantriol a également une action d'agent filmogène susceptible de recouvrir l'ongle, d'où une éventuelle protection supplémentaire. La composition du procédé selon l'invention est donc particulièrement remarquable par le fait qu'elle contient du phytantriol en tant qu'agent filmogène et/ou restructurant, voire hydratant, de l'ongle. On peut ajouter le phytantriol à raison de 0,01 à 0,5% en poids dans la composition finale.
La composition du procédé selon l'invention est préparée selon l'état de la technique.

Elle comprend généralement un polymère filmogène en solution organique, auquel on ajoute un plastifiant et éventuellement d'autres polymères, des agents rhéologiques et des épaississants.

Comme polymère filmogène, on peut utiliser la nitrocellulose, l'acétobutyrate de cellulose, les butyralpolyvinyliques, en solution à 5-25% dans des solvants tels que le toluène, le xylène, l'acétate d'éthyle, les esters et les alcools.
Comme plastifiant et autre polymère, on peut utiliser l'acétyl-tributyl-citrate, le dibutyl-phtalate, le camphre, les résines alkydes, les polyesters, les acryliques, les polyuréthannes, généralement en une quantité de 5-30%.

On peut ajouter à la composition du procédé selon l'invention des agents rhéologiques et/ou épaississants tels que la bentonite, les dérivés cellulosiques et les silices pyrogénées.

La composition peut également comprendre tout additif solubilisable ou dispersible dans le milieu solvant de la composition, et connu par l'homme du métier comme étant susceptible d'être incorporé dans une telle composition.
Ainsi, si l'on souhaite préparer une composition colorée, on peut ajouter des pigments, des laques, de la nacre et/ou des colorants usuels.

De plus, il est possible d'incorporer dans la composition ainsi obtenue, des agents actifs susceptibles de renforcer l'action du phytantriol, par exemple des agents susceptibles de traiter, protéger, nourrir, durcir, revitaliser et/ou hydrater ledit ongle.

On peut citer, par exemple, le butyl-formamide pour durcir, le D-panthénol pour nourrir et hydrater, les vitamines et leurs dérivés, la kératine et ses dérivés, la cystine, le chitosane et ses dérivés, les céramides, la biotine, les oligo-éléments, la glycérine, les hydrolysats de protéines, les phospholipides, les agents antifongiques et les agents anti-bactériens. Ces agents actifs peuvent être présents en une quantité de l'ordre de 0,01 à 5% en poids.
La composition du procédé selon l'invention se présente sous la forme d'une solution organique éventuellement épaissie.

On obtient ainsi une composition de soin susceptible de permettre l'obtention, après application pendant un certain temps de l'ordre de plusieurs semaines, d'un ongle restructuré, renforcé et mieux nourri et hydraté, présentant une tendance à se dédoubler et à se fendiller amoindrie, tout en ayant retrouvé une bonne souplesse.

L'invention est illustrée plus en détail dans les exemples suivants.

### Exemple 1

On prépare un vernis à ongles ayant la composition suivante, en matière sèche:
. toluène 30%
. acétate de butyle 31.6%
. isopropanol 8%
. acide organique 0,1%
. épaississant 1%
. nitrocellulose 11%
. camphre 1,5%
. dibutyl-phtalate 6%
. Santolite® 6%
. résine alkyde 4%
. phytantriol 0,2%
. pigments et nacre 0,6%

On obtient ainsi un vernis à ongles aisément applicable sur l'ongle et permettant l'obtention d'un film uniforme et brillant. L'ongle ainsi traité a tendance à moins se fendiller et/ou se dédoubler.

### Exemple 2

On prépare un vernis à ongles ayant la composition suivante, en matière sèche:
. toluène 30%
. acétate de butyle 31.3%
. isopropanol 8%
. acide organique 0,1%
. épaississant 1%
. nitrocellulose 11%
. camphre 1,5%
. dibutyl-phtalate 6%
. Santolite® 6%
. résine alkyde 4%
. phytantriol 0,15%
. D-panthénol 0.35%
. pigments et nacre 0,6%

On obtient ainsi un vernis à ongles aisément applicable sur l'ongle et permettant l'obtention d'un film uniforme et brillant.
Ce vernis constitue à la fois un produit de maquillage et un produit de soin pour les ongles, et permet de nourrir et d'hydrater l'ongle tout en réduisant les risques de dédoublement.

### Exemple 3

On prépare une base pour soin des ongles ayant la composition suivante:
. toluène 21,97%
. acétate de butyle 10%
. acétate d'éthyle 10%
. acétate de n-propyle 10%
. isopropanol 25%
. nitrocellulose 9%
. dibutyl-phtalate 2%
. Santolite® 3%
. butyralpolyvinylique 5%
. acétyl-tributyl-citrate 3%
. filtre UV 0,5%
. phytantriol 0,03%
. butyl-formamide 0,5%

On obtient ainsi une base de soin pour ongle, susceptible de le renforcer et de le restructurer.
Après une application 2 à 3 fois par semaine, pendant huit semaines, on constate que l'ongle semble renforcé et durci, et a moins tendance à se dédoubler et/ou se fendiller.

## Revendications

1. Procédé de soin des ongles comprenant l'application sur les ongles d'une composition comprenant du phytantriol dans un milieu solvant, **caractérisée par le fait que** ladite composition se présente sous la forme d'une solution organique et est appliquée telle quelle sur les ongles.

2. Procédé selon la revendication 1, **caractérisé par le fait que** le phytantriol est présent dans la composition à raison de 0,01-0,5% en poids.

3. Procédé selon l'une des revendications 1 ou 2, **caractérisé par le fait que** la composition comprend, en outre, un agent actif susceptible de renforcer l'action du phytantriol.

4. Procédé selon la revendication 3, **caractérisé par le fait que** l'agent actif est choisi dans le groupe constitué par les agents actifs susceptibles de traiter, protéger, nourrir, durcir, revitaliser et/ou hydrater l'ongle.

5. Procédé selon la revendication 3, **caractérisé par le fait que** l'agent actif est choisi parmi le butyl-formamide, le D-panthénol, les vitamines et leurs dérivés, la kératine et ses dérivés, la cystine, le chitosane et ses dérivés, les céramides, la biotine, les oligo-éléments, la glycérine, les hydrolysats de protéines, les phospholipides, les agents antifongiques et les agents anti-bactériens.

6. Procédé selon l'une des revendications 3 à 5, **caractérisé par le fait que** l'agent actif est présent à raison de 0,01 à 5% en poids.

7. Procédé selon l'une quelconque des revendications précédentes, **caractérisé par le fait que** la composition comprend un polymère filmogène.

8. Procédé selon l'une quelconque des revendications précédentes, **caractérisé par le fait que** le milieu organique comprend au moins un solvant organique choisi parmi le toluène, le xylène, les esters, les alcools.

9. Procédé selon l'une quelconque des revendications précédentes, **caractérisé par le fait que** la composition comprend au moins un additif choisi parmi les plastifiants, les agents rhéologiques, les pigments, les colorants.

10. Utilisation d'une composition comprenant du phytantriol dans un milieu solvant, la composition se présentant sous la forme d'une solution organique, pour restructurer l'ongle et/ou pour diminuer la faculté de dédoublement et/ou de fendillement des ongles.

11. Utilisation d'une composition comprenant du phytantriol et du D-panthénol, dans un milieu solvant, la composition se présentant sous la forme d'une solution organique, pour nourrir et/ou hydrater l'ongle.

12. Utilisation d'une composition comprenant du phytantriol et du butyl formamide, dans un milieu solvant, la composition se présentant sous la forme d'une solution organique, pour durcir l'ongle.

## Patentansprüche

1. Verfahren zur Pflege der Nägel, das umfaßt, auf die Nägel eine Zusammensetzung aufzutragen, die Phytantriol in einem Lösungsmittelmedium enthält, **dadurch gekennzeichnet, daß** die Zusammensetzung als organische Lösung vorliegt und als solche auf die Nägel aufgetragen wird.

2. Verfahren nach Anspruch 1, **dadurch gekennzeichnet, daß** das Phytantriol in der Zusammensetzung in einer Menge von 0,01 bis 0,5 Gew.-% vorliegt.

3. Verfahren nach einem der Ansprüche 1 oder 2, **dadurch gekennzeichnet, daß** die Zusammensetzung außerdem einen Wirkstoff enthält, der die Wirkung des Phytantriols verstärken kann.

4. Verfahren nach Anspruch 3, **dadurch gekennzeichnet, daß** der Wirkstoff unter den Wirkstoffen ausgewählt ist, die den Nagel behandeln, schützen, nähren, härten, revitalisieren und/oder hydratisieren können.

5. Verfahren nach Anspruch 3, **dadurch gekennzeichnet, daß** der Wirkstoff unter Butylformamid, D-Panthenol, Vitaminen und deren Derivaten, Keratin und seinen Derivaten, Cystin, Chitosan und seinen Derivaten, Ceramiden, Biotin, Spurenelementen, Glycerin, Proteinhydrolysaten, Phospholipiden, fungiziden Wirkstoffen und antibakteriellen Wirkstoffen ausgewählt ist.

6. Verfahren nach einem der Ansprüche 3 bis 5, **dadurch gekennzeichnet, daß** der Wirkstoff in einer Menge von 0,01 bis 5 Gew.-% vorliegt.

7. Verfahren nach einem der vorhergehenden Ansprüche, **dadurch gekennzeichnet, daß** die Zusammensetzung ein filmbildendes Polymer enthält.

8. Verfahren nach einem der vorhergehenden Ansprüche, **dadurch gekennzeichnet, daß** das organische Medium mindestens ein organisches Lösungsmittel enthält, das unter Toluol, Xylol, Estern und Alkoholen ausgewählt ist.

9. Verfahren nach einem der vorhergehenden Ansprüche, **dadurch gekennzeichnet, daß** die Zusammensetzung mindestens einen Zusatzstoff enthält, der unter den Weichmachern, Rheologiehilfsmitteln, Pigmenten und Farbstoffen ausgewählt ist.

10. Verwendung einer Zusammensetzung, die Phytantriol in einem Lösungsmittelmedium enthält, wobei die Zusammensetzung in Form einer organischen Lösung vorliegt, um den Nagel zu restrukturieren und/oder seine Fähigkeit zu vermindern, sich zu spalten und/oder rissig zu werden.

11. Verwendung einer Zusammensetzung, die Phytantriol und D-Panthenol in einem Lösungsmittelmedium enthält, wobei die Zusammensetzung in Form einer organischen Lösung vorliegt, um den Nagel zu nähren und/oder zu hydratisieren.

12. Verwendung einer Zusammensetzung, die Phytantriol und Butylformamid in einem Lösungsmittelmedium enthält, wobei die Zusammensetzung in Form einer organischen Lösung vorliegt, um den Nagel zu härten.

## Claims

1. Nailcare process, comprising the application to the nails of a composition comprising phytanetriol in a. solvent medium, **characterized in that** the said composition is in the form of an organic solution and is applied in unmodified form to the nails.

2. Process according to Claim 1, **characterized in that** the phytanetriol is present in the composition in a proportion of 0.01-0.5% by weight.

3. Process according to either of Claims 1 and 2, **characterized in that** the composition also comprises an active agent capable of reinforcing the action of the phytanetriol.

4. Process according to Claim 3, **characterized in that** the active agent is chosen from the group consisting of active agents capable of treating, protecting, nourishing, hardening, revitalizing and/or moisturizing the nails.

5. Process according to Claim 3, **characterized in that** the active agent is chosen from butylformamide, D-panthenol, vitamins and derivatives thereof, keratin and its derivatives, cystine, chitosan and its derivatives, ceramides, biotin, trace elements, glycerol, protein hydrolysates, phospholipids, antifungal agents and antibacterial agents.

6. Process according to one of Claims 3 to 5, **characterized in that** the active agent is present in a proportion of from 0.01% to 5% by weight.

7. Process according to any one of the preceding claims, **characterized in that** the composition comprises a film-forming polymer.

8. Process according to any one of the preceding claims, **characterized in that** the organic medium comprises at least one organic solvent chosen from toluene, xylene, esters and alcohols.

9. Process according to any one of the preceding claims, **characterized in that** the composition comprises at least one additive chosen from plasticizers, rheological agents, pigments and colorants.

10. Use of a composition comprising phytanetriol in a solvent medium, the composition being in the form of an organic solution, to restructure the nails and/or to reduce the tendency of the nails to split and/or crack.

11. Use of a composition comprising phytanetriol and D-panthenol, in a solvent medium, the composition being in the form of an organic solution, to nourish and/or moisturize the nails.

12. Use of a composition comprising phytanetriol and butylformamide, in a solvent medium, the composition being in the form of an organic solution, to harden the nails.
